# EUROPEAN PATENT SPECIFICATION

(11) **EP 1 254 121 B1**
(45) Date of publication and mention of the grant of the patent: **01.02.2006**
(21) Application number: 00903628.6
(22) Date of filing: 25.01.2000
(51) Int. Cl.: C07D 239/69

(54) **PREPARATION OF SULFONAMIDES**
HERSTELLUNG VON SULFONAMIDEN
PREPARATION DE SULFONAMIDES

(43) Date of publication of application: 06.11.2002
(73) Proprietor: F. HOFFMANN-LA ROCHE AG, 4070 Basel (CH)
(72) Inventor: DEHOFF, Bradley, S., Longmont, CO 80503 (US); HARRINGTON, Peter, J., Louisville, CO 80027 (US); KHATRI, Hiralal, N., Louisville, CO 80027 (US)
(74) Representative: Witte, Hubert
(86) International application number: PCT/EP2000/000556
(87) International publication number: WO 2001/055120

(56) References cited:
- EP-A- 0 526 708
- EP-A- 0 601 386
- EP-A- 0 743 307
- EP-A- 0 801 062
- EP-A- 0 852 226

## Description

### FIELD OF THE INVENTION

The present invention relates to a process for preparing ethylene glycol sulfonamide derivatives.

### BACKGROUND OF THE INVENTION

Sulfonamides of the formula: 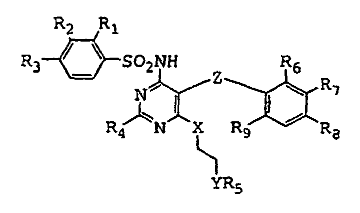
having a 1,2-diheteroethylene substituent (*i.e.,* an ethylene group containing heteroatom substituents on 1- and 2-positions) on the pyrimidine ring moiety, such as Bosentan, have a wide variety of biological activities including inhibiting the renin angiotensin system and acting as an endothelin antagonist. These compounds are useful in treatment of a variety of illnesses including cardiovascular disorders such as hypertension, ischemia, vasospasms and angina pectoris.

A current method of preparing ethylene glycol sulfonamide derivatives involves reacting an appropriately substituted pyrimidine monohalide with a monoanion ethylene glycol (*e.g*., sodium ethylene glycol) typically using ethylene glycol as a solvent. However, CS/19.01.2000 one of the disadvantages of using a monoanion of ethylene glycol is the formation of undesired ethylene glycol bis-sulfonamide in which two molecules of the pyrimidine monohalide are coupled with one molecule of ethylene glycol. The formation of this bis-sulfonamide compound requires costly and laborious separation steps to obtain a pharmaceutically suitable pure ethylene glycol sulfonamide compound. In addition, the use of ethylene glycol as a solvent, which is acceptable in a small scale reaction, is impracticable in a large industrial scale synthesis because of its toxicity and its high boiling point which requires a large amount of time and high energy consumption to remove it by distillation.

Another drawback to the current synthesis is the need for isolating a pyrimidine dihalide (W=halide) of the formula: 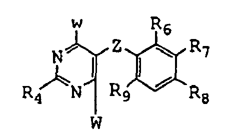
which is believed to be a potent sensitizer. This problem is further complicated by the use of a halogenated solvent, *e.g.,* methylene chloride, during the isolation of pyrimidine dihalide. Halogenated solvent is expensive to dispose of properly, thus leading to an added cost.

Furthermore, the current synthesis requires at least six separate isolation steps and the use of many different solvents, which makes it economically less attractive as an industrial process.

Therefore, there is a need for a process for preparing the above described 1,2-diheteroethylene sulfonamides with a reduced number of reaction product isolation steps. There is a need for a process for preparing the 1,2-diheteroethylene sulfonamides which does not produce undesired 1,2-diheteroethylene bis-sulfonamides. There is a need for a process for preparing the 1,2-diheteroethylene sulfonamides which does not require isolation of potent sensitizers such as pyrimidine dihalide and/or pyrimidine monohalide intermediates.

### SUMMARY OF THE INVENTION

The present invention provides a process for preparing a mono-protected 1,2-diheteroethylene substituted sulfon-amide of the formula: 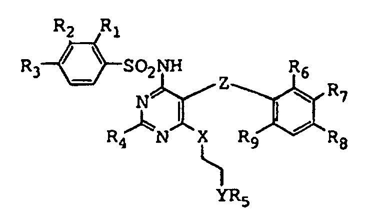
by contacting a pyrimidine monohalide of the formula: 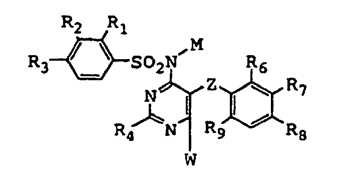
with a mono-protected 1,2-diheteroethylene anion of the formula M₁XCH₂CH₂YR₅, wherein
- R₁: is hydrogen, lower alkyl, lower alkoxy, lower alkylthio, halogen or trifluoromethyl;
- R₂: is hydrogen, halogen, lower alkoxy, trifluoromethyl or OCH₂COORₐ;
- R₃: is hydrogen, halogen, lower alkyl, lower alkylthio, trifluoromethyl, cycloalkyl, lower alkoxy or trifluoromethoxy; or
- R₂ and R₃: together can be butadienylene, methylenedioxy, ethylenedioxy or isopropylidenedioxy;
- R₄: is hydrogen, lower alkyl, cycloalkyl, trifluoromethyl, lower alkoxy, lower alkylthio, lower alkylthio-lower alkyl, hydroxy-lower alkyl, hydroxy-lower alkoxy, lower alkoxy-lower alkyl, hydroxy-lower alkoxy-lower alkyl, hydroxy-lower alkoxy-lower alkoxy, lower alkylsulfinyl, lower alkylsulfonyl, 2-methoxy-3-hydroxypropoxy, 2-hydroxy-3-phenylpropyl, amino-lower alkyl, lower alkylamino-lower alkyl, di-lower alkylamino-lower alkyl, amino, lower alkyl-amino, di-lower alkylamino, arylamino, aryl, arylthio, aryloxy, aryl-lower alkyl or heterocyclyl;
- R₅: is a protecting group;
- R₆, R₇, R₈ and R₉: are independently hydrogen, halogen, lower alkyl, trifluoromethyl, lower alkoxy, lower alkylthio, hydroxy, hydroxymethyl, cyano, carboxyl, formyl, methylsulfinyl, methylsulfonyl, methylsulfonyloxy or lower alkyloxy-carbonyloxy; or
- R₇: together with R₆ or R₈ can be butadienylene, methylenedioxy, ethylenedioxy or isopropylidenedi-oxy;
- Z: is O, S, ethylene, vinylene, C(=O), OCHR₁₀, or SCHR₁₀;
- R₁₀: is hydrogen or lower alkyl;
- X and Y: are independently O, S, or NH;
- M: is hydrogen, an alkaline metal or an alkaline earth metal;
- M₁: is an alkaline metal or an alkaline earth metal; and
- W: is a halide.

Preferably, the reaction is conducted in a nonpolar aprotic solvent. The present invention also provides a method for removing the protecting group, R₅.

In one aspect of the invention, X and Y are O and the protecting group, R₅, is a *tert-*butyl group which is used to protect the hydroxy group of ethylene glycol as an ether. The *tert*-butyl group of an ether is then removed using a formic acid to produce a formyloxy-protected ethylene glycol sulfonamide derivative (R₅ = CHO). Treatment of this compound with a base then produces an ethylene glycol sulfonamide derivative containing a free hydroxy group.

The present invention also provides a process for producing the pyrimidine monohalide by contacting a pyrimidine dihalide of the formula: 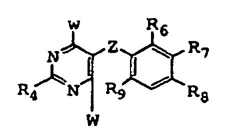
with a sulfonamide of the formula: 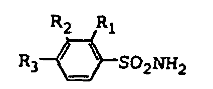
This coupling reaction is preferably conducted in a nonpolar solvent and is facilitated by the presence of a base and a phase transfer catalyst which increases the rate of reaction. It should be appreciated that when a base is present, the actual compound which is contacted with the pyrimidine dihalide may be the anion of the sulfonamide. By using the same reaction solvent in this reaction as the subsequent reaction, a need for isolation and/or purification of pyrimidine monohalide is avoided. As used herein, the term "isolation" of a compound refers to concentrating or separating the reaction product or a resulting work-up product such that the resulting composition, including any solvent that may be present, comprises at least about 80% of the compound, preferably at least about 90% of the compound, and more preferably at least about 95% of the compound. The term "purification" refers to a process for separating the desired compound from undesired compounds. A purity of a compound refers to the amount of the desired compound present in the mixture, not including any solvent which may also be present. Thus, a 90% pure compound dissolved in a large volume of solvent may still be considered to be 90% pure, but it may not be considered to be "isolated" since there is a large amount of solvent still present.

Isolation and/or purification of each product in the reaction is avoided by using a nonpolar solvent as the reaction solvent. Preferably the nonpolar solvent is an aprotic solvent, such as an ether and a hydrocarbon, more preferably the nonpolar solvent is selected from the group consisting of toluene, tetrahydrofuran and 2-methyltetrahydrofuran, and most preferably the nonpolar solvent is toluene.

Another aspect of the present invention is production of the pyrimidine dihalide by contacting a pyrimidinedione of the formula: 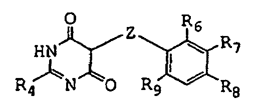
with a dehydrohalogenating agent. The product, pyrimidine dihalide, is a potent sensitizer, and the process of the present invention allows using the pyrimidine dihalide in the subsequent process without the need for isolation.

The present invention is particularly useful in the synthesis of an ethylene glycol sulfonamide derivative of the formula: 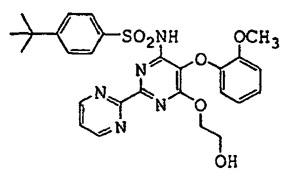
from the corresponding starting materials.

The present invention embraces a process for preparing an ethylene glycol sulfonamide of the formula: 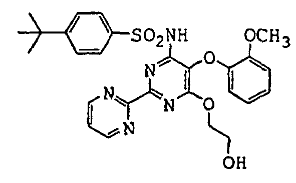
comprising:
(a) contacting a pyrimidinedione of the formula: 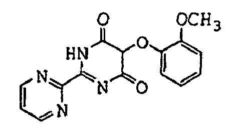
   with a dehydrohalogenating agent to produce a pyrimidine dihalide of the formula: 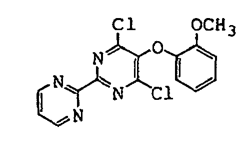
(b) contacting said pyrimidine dihalide with a sulfonamide of the formula: 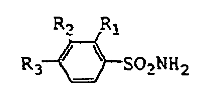
   in a nonpolar aprotic solvent in the presence of a first base and a phase transfer catalyst to produce a pyrimidine monohalide of the formula: 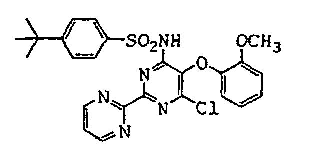
(c) contacting said pyrimidine monohalide with a mono-protected ethylene glycol of the formula HOCH₂CH₂OR₅ in said nonpolar aprotic solvent in the presence of a second base to produce a mono-protected ethylene glycol sulfonamide of the formula: 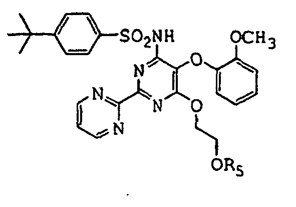
   wherein
   R₅ is a hydroxy protecting group; and
(d) removing the protecting group to produce said ethylene glycol sulfonamide.

Another embodiment of the present invention provides new chemical compounds p-*tert*-butyl-N-[6-(2-*tert*-butoxyethoxy)-5-(o-methoxyphenoxy)-2-(pyrimidin-2-yl)-pyrimidin-4-yl] benzenesulfonamide, p-*tert*-butyl-N-[6-(2-formyloxyethoxy)-5-(o-methoxyphenoxy)-2-(pyrimidin-2-yl)-pyrimidin-4-yl] benzenesulfonamide, p-*tert*-butyl-N-[6-(2-formyloxyethoxy)-5-(o-methoxyphenoxy)-2-(pyrimidin-2-yl)-pyrimidin-4-yl] benzenesulfonamide monoethyl alcohol solvate in a crystalline form and p-*tert*-butyl-N-[6-chloro-5-(o-methoxyphenoxy)-2-(pyrimidin-2-yl)-pyrimidin-4-yl] benzenesulfonamide potassium salt.

### BRIEF DESCRIPTION OF THE DRAWINGS

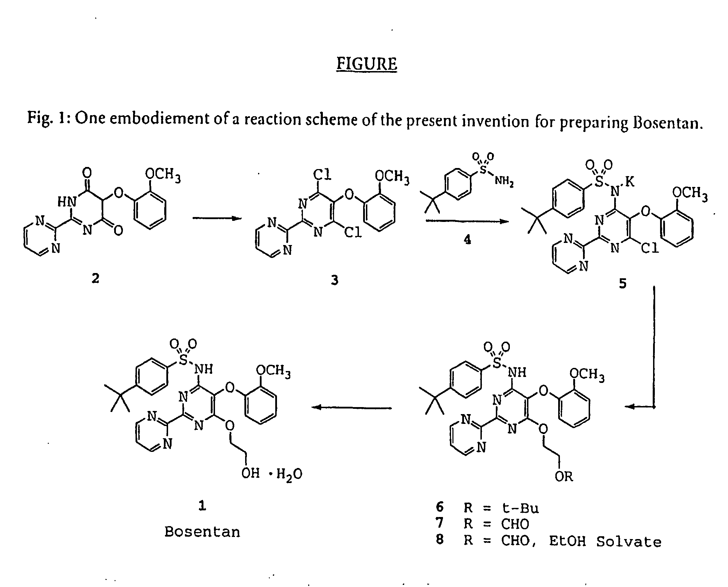

Figure 1 shows one embodiment of a reaction scheme of the present invention for preparing Bosentan.

### DETAILED DESCRIPTION OF THE INVENTION

The present invention provides a process for preparing mono-protected 1,2-diheteroethylene sulfonamide of the formula I and their hydrates: 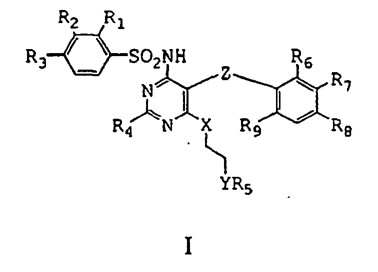
wherein
- R₁: is hydrogen, lower alkyl, lower alkoxy, lower alkylthio, halogen or trifluoromethyl;
- R₂: is hydrogen, halogen, lower alkoxy, trifluoromethyl or OCH₂COORₐ;
- R₃: is hydrogen, halogen, lower alkyl, lower alkylthio, trifluoromethyl, cycloalkyl, lower alkoxy or trifluoromethoxy; or
- R₂ and R₃: together can be butadienylene, methylenedioxy, ethylenedioxy or isopropylidenedioxy;
- R₄: is hydrogen, lower alkyl, cycloalkyl, trifluoromethyl, lower alkoxy, lower alkylthio, lower alkylthio-lower alkyl, hydroxy-lower alkyl, hydroxy-lower alkoxy, lower alkoxy-lower alkyl, hydroxy-lower alkoxy-lower alkyl, hydroxy-lower alkoxy-lower alkoxy, lower allcylsulftnyl, lower alkylsulfonyl, 2-methoxy-3-hydroxypropoxy, 2-hydroxy-3-phenylpropyl, amino-lower alkyl, lower alkylamino-lower alkyl, di-lower alkylamino-lower alkyl, amino, lower alkyl-amino, di-lower allrylamino, arylamino, aryl, arylthio, aryloxy, aryl-lower alkyl or heterocyclyl;
- R₅: is a protecting group;
- R₆, R₇, R₈ and R₉: are independently hydrogen, halogen, lower alkyl, trifluoromethyl, lower alkoxy, lower alkylthio, hydroxy, hydroxymethyl, cyano, carboxyl, formyl, methylsulfinyl, methylsulfonyl, methylsulfonyloxy or lower alkyloxy-carbonyloxy; or
- R₇: together with R₆ or R₈ can be butadienylene, methylenedioxy, ethylenedioxy or isopropylidenedi-oxy;
- Z: is O, S, ethylene, vinylene, C(=O), OCHR₁₀, or SCHR₁₀;
- R₁₀: is hydrogen or lower alkyl; and
- X and Y: are independently O, S, or NH;

and salts thereof.

The process of the present invention provides many advantages and improvements over the current processes of synthesizing the above defined 1,2-diheteroethylene sulfonamide compounds. The corresponding starting materials are either commercially available or can be obtained by the processes described in the European Patent Application EP 0 526 708.

The term "lower", as used herein, denotes groups with 1-7 carbon atoms, preferably 1-4 carbon atoms. Alkyl, alkoxy and allcylthio groups as well as alkyl groups as components of alkanoyl groups can be straight-chain or branched. Methyl, ethyl, propyl, isopropyl, butyl, *sec*- and *tert*-butyl are examples of such alkyl groups. Halogen denotes fluorine, chlorine, bromine and iodine, with chlorine being preferred. Cycloalkyl denotes residues with 3 to 8 carbon atoms, such as cyclopropyl, cyclobutyl, cyclopentyl or cyclohexyl, and the like. Examples of aryl residues are phenyl and substituted phenyl residues, with halogen, lower alkyl, lower alkoxycarboxyl and trifluoromethyl especially coming into consideration as substituents. Examples of heterocyclyl residues are unsubstituted or, preferably, substituted (for example, mono- or disubstituted with lower alkyl, lower alkoxy, halogen, aryl, aryl-lower alkyl or mixtures thereof) mono- or bicyclic 5- and 6-membered heterocyclic residues with oxygen, nitrogen or sulfur as the hetero atom, such as, for example, 2- and 3-furyl, pyrimidinyl, 2-, 3- and 4-pyridyl and pyridyl N-oxide, 1,2- and 1,4-diazinyl, morpholino, 2- and 3-thienyl, isoxazolyl, oxazolyl, imidazolyl, pyrrolyl, benzofuranyl, benzothienyl, indolyl, purinyl, quinolyl, isoquinolyl, quinazolyl, and the like.

With respect to compound I:
Preferably, Z is O and, furthermore, R₆ is lower alkoxy, especially methoxy, and R₇, R₈ and R₉ are hydrogen.
R₅ is a protecting group. It will be recognized that the identity of the protecting group depends on the identity of the Y moiety. Thus, for example, when Y is NH then R₅ is an amine protecting group, when Y is S then R₅ is a thiol protecting group and when Y is O then R₅ is a hydroxy protecting group. Suitable protecting groups for a given Y moiety are well known to one of ordinary skill in the art, and some of the representative suitable protecting groups are disclosed in "Protecting Groups in Organic Synthesis," T.W. Greene, John Wiley & Sons, New York, N.Y., 1981, which is incorporated herein in its entirety. Preferably, when X and Y are O, R₅ is *tert*-butyl.
R₁ and R₂ are preferably hydrogen.
R₃ is preferably lower alkyl, more preferably *t*-butyl.
R₄ is preferably 2-pyrimidinyl.
X and Y are preferably oxygen.

The process of the present invention includes contacting a pyrimidine monohalide of the formula II: 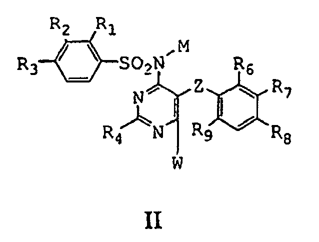
with a mono-protected 1,2-diheteroethylene anion of the formula M₁XCH₂CH₂YR₅ to produce the mono-protected 1,2-diheteroethylene sulfonamide I. M is hydrogen or a metal, preferably hydrogen, an alkali metal or alkali-earth metal, and more preferably hydrogen or an alkali metal. Still more preferably M is selected from the group consisting of hydrogen, sodium, lithium and potassium, and most preferably M is selected from the group consisting of hydrogen, sodium, and potassium. M₁ is a metal, preferably an alkali metal or alkali-earth metal, more preferably an alkali metal. Still more preferably M₁ is selected from the group consisting of lithium, potassium and sodium, and most preferably M₁ is sodium. A preferred reaction temperature is from about 15 °C to about 100°C, more preferably from about 30 °C to about 80 °C, and most preferably from about 50 °C to about 60 °C. A preferred reaction time is from about 1 to about 15 hours, more preferably from about 2 to about 10 hours, and most preferably from about 3 to about 7 hours. Preferably from about 1 equivalents (eq.) to about 10 eq. of mono-protected 1,2-diheteroethylene anion relative to the pyrimidine monohalide II is used in the reaction, more preferably from about 1 eq. to about 5 eq., and most preferably from about 1 eq. to about 1.2 eq.

The mono-protected 1,2-diheteroethylene anion can be prepared prior to being added to the pyrimidine monohalide II, or it can be generated *in situ* by contacting the compound of the formula HXCH₂CH₂YR₅ with a base. It will be appreciated that any base which can deprotonate HXCH₂CH₂YR₅ can be used. Preferably the base is selected from the group consisting of hydroxides such as sodium hydroxide, calcium hydroxide, magnesium hydroxide, potassium hydroxide and lithium hydroxide; hydrides such as sodium hydride, potassium hydride, lithium hydride and calcium hydride; metals such as sodium; carbonates such as potassium carbonate, sodium carbonate, and lithium carbonate; alkoxides such as *tert*-butoxide, and isopropoxide; and bicarbonates such as lithium bicarbonate, sodium bicarbonate, and potassium bicarbonate; and mixtures thereof. More preferably the base is a hydroxide, still more preferably the base is selected from the group consisting of sodium hydroxide, potassium hydroxide, calcium hydroxide, magnesium hydroxide and lithium hydroxide, and most preferably the base is sodium hydroxide.

The preparation of mono-protected 1,2-diheteroethylene sulfonamide I can be carried out in the absence of substantially any solvent or it can be carried out in the presence of a reaction solvent. As used in this invention, "absence of substantially any solvent" means that the amount of solvent present is less than about 5% by volume (L) per kg of compound I (% vol/wt), preferably less than about 2% vol/wt, and more preferably less than about 1% vol/wt.

When the reaction solvent is present, it is preferred that the reaction solvent is a nonpolar solvent. As used in this invention a "nonpolar solvent" refers to a solvent having a dielectric constant of less than about 20, preferably less than about 15, more preferably less than about 10, and most preferably less than about 5. As used in this invention, the dielectric constant, ε, of a solvent refers to the value at 20 °C. The dielectric constant of a solvent can be found, for example, in Handbook of Chemistry and Physics, 63^{rd} Ed., CRC Press, 1983, pp. E-51 to E-54, which is incorporated herein by reference. More preferable the reaction solvent is aprotic solvent, such as ethers and hydrocarbons. As used herein, an "aprotic solvent" refers to a solvent which is not a good hydrogen bond donor, *i.e.,* solvents that do not contain heteroatom-hydrogen bond, *e.g.,* O-H, or N-H bond. It should be appreciated, however, that while aprotic solvents are not good hydrogen bond donors, they may or may not be good hydrogen bond acceptors. Still more preferably the reaction solvent is selected from the group consisting of toluene, tetrahydrofuran and 2-methyltetrahydrofuran, and most preferably the reaction solvent is toluene.

The mono-protected 1,2-diheteroethylene sulfonamide I can be isolated from the reaction mixture by adding a sufficient amount of acid to the reaction mixture to neutralize any base that may be present, removing the reaction solvent and crystallizing or precipitating it from a crystallization solvent. Preferably, a sufficient amount of acid is added to the reaction mixture resulting in the pH of the solution from pH of about 5 to pH of about 7, more preferably, from pH of about 5 to pH of about 6, and most preferably from pH of about 5 to pH of about 5.5. Any acid having sufficient pKa to generate the desired pH can be used. Preferably the acid is selected from the group consisting of inorganic acids such as hydrochloric acid, hydriodic acid, hydrobromic acid, phosphoric acid and sulfuric acid, and more preferably the acid is hydrochloric acid. Preferably, the crystallization solvent is a lower alkyl alcohol, and more preferably ethanol. Preferably the crystallization solvent is maintained at a temperature of from about -25°C to about 50°C to crystallize the reaction product, more preferably from about -10°C to about 25°C, and most preferably from about -5 °C and 10°C.

Unlike the process described in the Background section, one particular embodiment of the present invention provides a process for preparing a mono-protected ethylene glycol sulfonamide derivative of compound I (where X and Y are O) using a mono-protected ethylene glycol derivative which prevents formation of an undesired ethylene glycol bis-sulfonamide compound, *e.g*., where two molecules of compound II are coupled to one molecule of ethylene glycol to form a molecule of a general structure Ar-OCH₂CH₂O-Ar, wherein Ar is the portion of the compound II which has been coupled to ethylene glycol. Without being bound by any theory, it is believed that in the process described in the Background section, the hydroxy group of some of the initially formed ethylene glycol sulfonamide derivative reacts with unreacted sodium ethylene glycol (NaOCH₂CH₂OH) or other bases which may present in the reaction mixture to form an anion which then reacts with another molecule of pyrimidine mono-halide II to produce the undesired ethylene glycol bis-sulfonamide derivative. By using the mono-protected ethylene glycol derivative, the present invention eliminates any possibility of forming such an anion, thus completely eliminating production of the undesired ethylene glycol bis-sulfonamide derivative. This elimination of the production of undesired bis-sulfonamide ethylene glycol derivative results in higher overall product yield and easier product purification.

Another shortcoming in the process described in the Background section is the use of ethylene glycol as a solvent which must be removed by distillation after the reaction. In a small scale reaction, using ethylene glycol as a solvent does not pose much difficulty. In a large industrial scale reaction, however, using ethylene glycol is impracticable because of its toxicity and its high boiling point which requires a large amount of time and energy for its removal. In contrast, the process of the present invention uses a nonpolar solvent as discussed above.

The process for preparing the mono-protected 1,2-diheteroethylene sulfonamide I can further include a step of removing the protecting group, *i*.*e*., conversion of R₅ to hydrogen. Removal of a variety of protecting groups is disclosed in the above mentioned "Protecting Groups in Organic Synthesis."

As an illustration, the process for removing a protecting group of mono-protected ethylene glycol sulfonamide I will be discussed with regard to removing a *tert*-butyl ether protecting group of an alcohol (*i*.*e*., conversion of R₅ from *tert*-butyl to hydrogen, where X and Y are O). Contacting the *tert-butyl* ether protected ethylene glycol sulfonamide I (i.e., compound I wherein YR₅ is a O-*tert*-butyl moiety) with an acid removes the *tert*-butyl protecting group. Any acid having a sufficient acidic strength to remove *tert*-butyl ether group can be used. Exemplary acids include organic acids such as toluenesulfonic acid, trifluoroacetic acid (TFA), methanesulfonic acid (MSA), formic acid, acetic acid and other carboxylic acids; inorganic acids such as sulfuric acid, hydrobromic acid, hydriodic acid and hydrochloric acid; and Lewis acids such as ZnCl₂, AlCl₃, FeCl₃, TiCl₄, and Me₃SiI. Such acids can be used individually or as a mixture. Preferably the acid is selected from the group consisting of trifluoroacetic acid (TFA), methanesulfonic acid (MSA), formic acid, acetic acid, sulfuric acid, hydrochloric acid, FeCl₃, TiCl₄, and Me₃SiI, and more preferably the acid is formic acid.

When using a protic acid in the deprotection of an ether protecting group, it is preferred that an alcohol solvent is used in the deprotection reaction. As used in this invention, the term "protic acid" refers to a Bronsted-Lowry acid, *i.e.,* any substance that is capable of giving up a hydrogen ion, or proton. Preferably, the alcohol solvent is selected from the group consisting of methanol, ethanol, iso-propanol and butanol, more preferably the alcohol solvent is selected from the group consisting of methanol, ethanol and iso-propanol, and most preferably the alcohol solvent is ethanol.

With regards to *tert*-butyl ether protected ethylene glycol sulfonamide I, a useful reaction temperature for the deprotection of an ether is from about 10 °C to about 125 °C, more preferably from about 25 °C to about 100 °C, and most preferably from about 80 °C to about 90 °C. The ratio of the protic acid to the *tert*-butyl ether protected ethylene glycol sulfonamide I can be from about 1 liter of the acid:1 kilogram of the mono-protected compound (*i.e*., 1:1 (1/kg)) to about 10:1 (1/kg), preferably about 5:1 (1/kg), and most preferably about 2:1 (1/kg). Under these conditions, less than about 5% of residual *tert-*butyl ether protected ethylene glycol sulfonamide I remains after about 1 to 10 hours, and preferably less than about 1%. Preferably, the deprotection reaction results in less than about 1% of *tert*-butyl ether protected ethylene glycol sulfonamide I remaining.

After the deprotection reaction, the reaction mixture is cooled and a nonpolar aprotic solvent, as discussed above, is added. A substantial amount of nonpolar solvent and the protic acid is then removed, for example, by azeotropic distillation under a reduced pressure.

When formic acid is used for the deprotection of *tert*-butyl ether protected ethylene glycol sulfonamide I, the initial product can be a formyloxy-protected ethylene glycol sulfonamide I (*i.e.,* compound I, wherein X and Y are O and R₅ is CHO). The invention therefore also relates to a process wherein contacting a mono-protected 1,2-diheteroethylene sulfonamide with formic acid produces an intermediate mono-protected 1,2-diheteroethylene solfonamide of the formula:

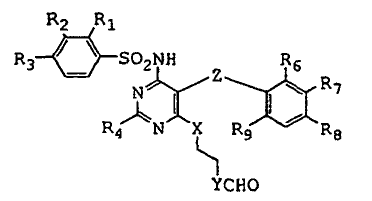

The formyloxy-protected ethylene glycol sulfonamide I is typically isolated from the reaction mixture by the following process. The reaction mixture containing formyloxy-protected ethylene glycol sulfonamide I is cooled to from about 25°C to about 100 °C, more preferably from about 35 °C to about 85 °C, and most preferably to from about 45 °C to about 55 °C. The resulting slurry is then diluted with an alcohol solvent, preferably ethanol, and heated to reflux. Cooling the resulting alcohol solvent mixture to from about -25 °C to about 25 °C, preferably from about -15 °C to about 15 °C, and more preferably from about -10 °C to about 0 °C, affords the desired formyloxy-protected ethylene glycol sulfonamide I. In some cases, a solvated formyloxy-protected ethylene glycol sulfonamide I is obtained by this process, *i.e.,* a solid formyloxy-protected ethylene glycol sulfonamide I containing solvent molecules, e.g., ethanol. The term "solvated" means a solid compound which contains solvent molecules within the crystal lattice of the compound.

Alternatively, the alcohol solvent mixture from above is cooled to from about 0 °C to about 50 °C, more preferably from about 15 °C to about 35 °C, and most preferably to about 25 °C. The solvent is removed by decantation from a crystallized slurry containing product. Although the product may be dried, typically the wet product is used directly in the subsequent process.

The formyloay group can then be removed by contacting the formyloxy-protected ethylene glycol sulfonamide with a base. Any base which can hydrolyze the formyloxy group can be used. Preferably the base is selected from the group consisting of hydroxides such as sodium hydroxide, potassium hydroxide, lithium hydroxide, calcium hydroxide and magnesium hydroxide; carbonates such as sodium carbonate, lithium carbonate, potassium carbonate and calcium carbonate; bicarbonates such as sodium bicarbonate, potassium bicarbonate and lithium bicarbonate. More preferably the base is selected from the group consisting of hydroxide, and most preferably sodium hydroxide. The deprotection of the formyloxy group can be performed in the presence of a solvent. Preferably, the solvent is a protic solvent such as water, alcohol and a mixture thereof, more preferably the solvent is water, ethanol and a mixture thereof.

For removal of the formyloxy group, typically the combined mixture is stirred at a temperature of from about 5 °C to about 65°C, more preferably from about 15 °C to about 45°C, and most preferably at about 25 °C. The reaction time can range from about 5 minutes to about 48 hours, more preferably from about 15 minutes to about 5 hours, and most preferably from about 30 minutes to about 90 minutes. After the removal of formyloxy group, the reaction mixture is acidified to adjust the pH of the reaction mixture to pH of from about 5 to pH of about 7, more preferably to pH of from about 5 to pH of about 6, and most preferably to pH of from about 5 to pH of about 5.5. Any acid which is sufficiently strong enough to adjust the pH range of the reaction mixture to a desired pH can be used. Preferably the acid is hydrochloric acid, more preferably the acid is 12 N HCI solution. After the addition of acid, water is added and the suspension is stirred for from about 1 hour to about 10 hours, more preferably from about 2 hours to about 5 hours, and most preferably for about 3 hours. The solid product, ethylene glycol sulfonamide (*i.e*., compound I, wherein X and Y are O and R₅ is hydrogen), is then filtered, washed with an alcohol-water mixture, preferably ethanol-water mixture, and dried using standard process to afford the desired ethylene glycol sulfonamide.

The ethylene glycol sulfonamide can be further purified by refluxing the solution of the wet impure ethylene glycol sulfonamide in an alcohol, preferably ethanol, with the addition of water during the reflux. The resulting suspension is then cooled to a range from about 0 °C to about 50°C, more preferably from about 15 °C to about 35°C, most preferably from about 20 °C to about 30 °C. The mixture is cooled to a desired temperature over a period of from about 1 hour to about 24 hours, more preferably from about 2 hours to about 12 hours, and most preferably from about 5 hours to about 7 hours. The purified ethylene glycol sulfonamide is then isolated and dried. Using this process, ethylene glycol sulfonamide having a purity of greater than about 99.3% can be produced, more preferably greater than about 99.5%, and most preferably greater than about 99.8%.

The process for preparing mono-protected 1,2-diheteroethylene sulfonamide I of the present invention can also include a process for preparing the pyrimidine monohalide II by contacting a pyrimidine dihalide III of the formula: 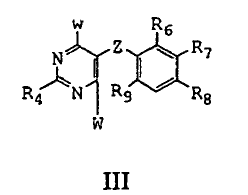
with a sulfonamide IV of the formula: 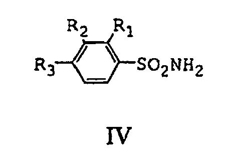
This coupling reaction between the pyrimidine dihalide III and the sulfonamide IV can include the presence of a base. Without being bound by any theory, it is believed that the base deprotonates the sulfonamide and neutralizes any acid that is generated during the reaction. It is believed that in the absence of a base, the resulting acid that is generated in the coupling reaction can reduce the rate of subsequent coupling reaction by protonating the sulfonamide IV thereby reducing its reactivity, or the acid can cause degradation of the product and/or starting material resulting in a decrease in the overall yield. It should be appreciated that in the presence of a base, the reactive species may be the deprotonated sulfonamide (*i.e.,* sulfonamide anion). Thus, while the sulfonamide is represented in its neutral form, in the presence of a base, the process of the present invention also encompasses the corresponding sulfonamide anion. Preferably, the base is selected from the group consisting of sodium bicarbonate, potassium bicarbonate, lithium bicarbonate, sodium carbonate, potassium carbonate, lithium carbonate, sodium hydroxide, lithium hydroxide, calcium hydroxide, magnesium hydroxide and potassium hydroxide, more preferably the base is selected from the group consisting of potassium carbonate, sodium carbonate, sodium hydroxide and potassium hydroxide, and most preferably the base is potassium carbonate. The amount of base used in this reaction is from about 1 eq. to about 2 eq., preferably from about 1 eq. to about 1.5 eq., more preferably from about 1 eq. to about 1.3 eq., and most preferably about 1.1 eq.

This coupling reaction can be conducted in the same reaction solvent as the solvent used in the coupling reaction between the pyrimidine monohalide II and the mono-protected 1,2-diheteroethylene compound. Moreover, the reaction mixture of the coupling reaction between the pyrimidine dihalide III and the sulfonamide IV can be used directly in the next step without isolation or purification.

The coupling reaction between the pyrimidine dihalide III and the sulfonamide IV can also include the presence of a phase transfer catalyst. A "phase transfer catalyst" refers to a catalyst or agent which is added to a reaction mixture of components, to transfer one or more of the reacting components to a location where it can conveniently and rapidly react with another reacting component. Non-limiting examples of phase transfer catalysts or agents that may be employed are reviewed in "Phase-Transfer Catalysis," by C.M. Starks et al., Chapman & Hall, New York, N.Y., 1994, which is incorporated herein by reference in its entirety. Preferably the phase transfer catalyst is selected from the group consisting of tetrabutylammonium bromide, tetrabutylphosphonium bromide, tetrabutylammonium chloride, tetrabutylphosphonium chloride, benzyltriethylammonium chloride, and tetrabutylammonium hydrogen sulfate, and more preferably tetrabutylammonium bromide. Preferably from about 0.5 mole% to about 10 mole% of phase transfer catalyst is added to the reaction mixture, more preferably from about 1 mole% to about 5 mole%, still more preferably from about 1.5 mole% to about 2.5 mole%, and most preferably about 2 mole%.

Preferably the reaction time is from about 2 hours to about 15 hours, more preferably from about 5 hours to about 10 hours, and most preferably from about 5 hours to about 7 hours. The reaction can be conducted under a condition where any water that is present or formed in the reaction mixture is removed. For example, this can be achieved by using a reaction solvent which can remove water azeotropically using a Dean-Stark apparatus. Preferably the reaction solvent is same as the solvent used in the coupling reaction between the pyrimidine monohalide II and the mono-protected 1,2-diheteroethylene compound. Use of the same reaction solvent allows the coupling reaction between the pyrimidine monohalide II and the mono-protected 1,2-diheteroethylene compound to be conducted without isolating the product from the coupling reaction between the pyrimidine dihalide III and the sulfonamide IV. This elimination of a need for isolation of a product reduces the production time and overall cost. Moreover, it eliminates isolation of sensitizer pyrimidine monohalide II, thus reducing the risk of exposure to a harmful chemical.

The process for preparing mono-protected 1,2-diheteroethylene sulfonamide I of the present invention can also include a process for preparing a pyrimidine dihalide III by contacting pyrimidinedione V of the formula: 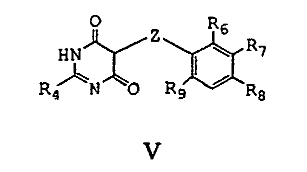
with a dehydrohalogenating reagent. As used in this invention, a "dehydrohalogenating reagent" refers to any reagent which is capable of converting the pyrimidinedione V to pyrimidine dihalide III. Exemplary dehydrohalogenating reagents include phosphorus oxychloride, phosphorous pentachloride, phosphorous trichloride, phosphorus oxybromide, phosphorous pentabromide, phosphorous tribromide, oxalyl chloride, and mixtures thereof. Preferably the dehydrohalogenating agent is selected from the group consisting of phosphorous oxychloride, phosphorous pentachloride, phosphorous trichloride, and mixtures thereof.

The conversion of pyrimidinedione V to pyrimidine dihalide III using a dehydrohalogenating agent is typically conducted at an elevated temperature. Preferably the reaction temperature is at least about 80 °C, more preferably at least about 85 °C, and most preferably at least about 90 °C. Although the reaction can be conducted in any solvent which is substantially inert to the reaction conditions, typically the reaction is conducted in the absence of a solvent. After a sufficient amount of the pyrimidine dihalide III is formed, the reaction mixture is diluted with a solvent which has a boiling point of at least 80 °C, preferably at least about 90 °C, and more preferably at least about 110°C. Preferably the solvent is a nonpolar solvent, more preferably an aprotic nonpolar solvent, still more preferably toluene, and most preferably the same nonpolar solvent as that used in the subsequent process. By using the same nonpolar solvent as the subsequent process, the present invention avoids having to isolate and/or purify the pyrimidine dihalide III which is a known to be a potent sensitizer. The resulting reaction mixture is then quenched to destroy any remaining dehydrohalogenating agent. The quenching agent is any compound which reacts with the dehydrohalogenating agent without significantly reacting with pyrimidinedione V and/or pyrimidine dihalide III. Preferably the quenching agent is selected from an alcohol, water, and mixtures thereof. More preferably the quenching agent is water. The quenching agent can also contain a base to neutralize any acid that may be formed during the quenching step. Any base which can neutralize the acid that is formed in the quenching step can be used. Preferably the base is a hydroxide, and more preferably sodium hydroxide.

When a phosphorous compound is used as the dehydrohalogenating agent, phosphorous by-products are produced during the quenching step. The removal of these phosphorous by-products can be facilitated by adding a metal oxide. Preferably the metal oxide is selected from the group consisting of a transition metal oxide, alkali-earth metal oxide and alkali metal oxide, more preferably the metal oxide is selected from the group consisting of an alkaline-earth metal oxide, and most preferably the metal oxide is calcium oxide. The pyrimidine dihalide III can be isolated from the reaction mixture prior to the subsequent process; however, the pyrimidine dihalide III is believed to be a sensitizer. Therefore, it is preferred that the pyrimidine dihalide III be used in the subsequent process without first being isolated or purified.

Additional objects, advantages, and novel features of this invention will become apparent to those skilled in the art upon examination of the following examples thereof, which are not intended to be limiting.

### EXAMPLES

4-*tert*-Butylbenzenesulfonamide (4) was purchased from Saurefabrik Schweizerhall. Phosphorus oxychloride, potassium carbonate, tetrabutylammonium bromide, sodium hydroxide beads, and formic acid were purchased from Aldrich Chemical Company. Toluene was purchased from Burdick and Jackson. Ethylene glycol mono *tert*-butyl ether (ETB) was purchased from TCI America. Ethanol was purchased from Spectrum Chemical.

Ethylene glycol mono-*tert*-butyl ether is also available from Maruzen, and Spectrum.

All other reagents and solvents are readily commercially available, for example from Aldrich Chemical Company or equivalent commercial suppliers.

### Example 1

This example illustrates the preparation of 4,6-dichloro-5-(O-methoxy-phenoxy)-2,2'-bipyrimidine (3).

A mixture of 150.0 g (0. 480 mol) of 5-(O-methoxy-phenoxy)-2-(2-pyrimidin-2-yl)-4,6 (1H,5H)-pyrimidine dione (2) and 176 mL (290 g, 1.89 mol) phosphorus oxychloride was heated to 90 °C. After the vigorous gas evolution subsided, the pot temperature was increased to 105 °C and maintained there for 5 h. The mixture was cooled to 80-90 °C, diluted with 225 mL toluene then added via a 12 gauge cannula to a mixture of 675 mL toluene and 525 mL H₂O over 15-30 min. External cooling is used to maintain the quench mixture temperature at less than 80 °C. Aqueous sodium hydroxide (400 mL of 30%) was added at 70-80 °C then the layers were separated. The toluene layer was washed with 500 mL of water containing 1 mL of 30% aq NaOH. To avoid precipitation of dichloropyrimidine (3) the temperature must be kept above 70 °C after the caustic addition.

The combined aqueous layers were extracted with 500 mL toluene. The combined organic phases were dried by distillation of the toluene azeotrope. The resulting solution was used directly in the next step.

### Example 2

This example illustrates the preparation of p-*tert*-butyl-N-[6-chloro-5-(O-methoxy-phenoxy) [2,2'-bipyrimidin]-4-yl] benzenesulfonamide potassium salt (5) using BTEAC.

A mixture of 6.427 g (30.13 mmol) of 4-tert-butylbenzenesulfonamide (4), anhydrous potassium carbonate (Armand, extra fine grade, 4.997 g, 36.16 mmol, 1.2 equiv.), 69 mg (0.301 mmol, 1 mole%) benzyltriethylammonium chloride (BTEAC), dichloropyrimidine (3) (10.521 g, 30.13 mmol) and toluene (150 mL) was refluxed (heating bath at 130°C, Dean-Stark trap) for 8 hours. The resulting mixture was cooled overnight.

To the stirred mixture was added 3.5 mL 12 N HCI (42 mmol) and water was removed by azeotropic distillation. Reaction analysis by HPLC showed 96.4% of the desired product after the acid work-up.

### Example 3

This example illustrates the preparation of p-*tert*-butyl-N-[6-chloro-5-(O-methoxy-phenoxy) [2,2'-bipyrimidin]-4-yl] benzenesulfonamide potassium salt (5) using TBAB.

4-*tert*-Butylbenzenesulfonamide (4) (102.4 g, 0.480 mol), 79.6 g (0. 576 mol) anhydrous powdered (extra fine) potassium carbonate, 4.6 g (14 mmol, 2.9 mol%) tetrabutylammonium bromide (TBAB), and 1950 mL toluene were added to the toluene solution of dichloropyrimidine (3) at 50 °C. The resulting suspension was refluxed with continuous removal of water using a Dean-Stark trap for 5-7 h. The suspension was cooled then used directly in the next step.

### Example 4

This example illustrates the preparation of p-*tert*-butyl-N-[6-(2-tert-butyloxy-ethoxy)-5-(O-methoxy-phenoxy) [2,2'-bipyrimidin]-4-yl] benzenesulfonamide (6).

Ethylene glycol mono-*tert*-butyl ether (ETB) (189 mL, 170 g, 1.44 mol) and 38.4 g (0.960 mol) of granular sodium hydroxide were added to the benzenesulfonamide potassium salt (5) suspension in toluene. The suspension was then heated at 55 °C for 3 to 7 h. The mixture changed from a slurry to a near solution to a suspension as the product precipitated near the end of the reaction. The suspension was cooled and 80 mL of 12 N HCI in 720 mL water was added. More acid (10-15 mL) was added to adjust the pH to 3-4 and produce two clear layers. The layers were separated. The organic layer was washed twice with 500 mL water.

The toluene-water azeotrope and toluene were distilled at atmospheric pressure (3,200 mL collected). The flask was cooled and distillation was continued under reduced pressure until approximately 50 mL of toluene remained. The pot solution was cooled and diluted with 1500 mL denatured ethanol. Toluene was removed as the ethanol azeotrope (500-750 mL collected) and the suspension allowed to cool to 25 °C overnight. After cooling to 2-5 °C, the suspension was stirred for 2 h. The precipitate was suction filtered, washed with 500 mL cold denatured ethanol, then dried in a vacuum oven at 40-50 °C to afford 268 g (91.8%) of near colorless powder.

Recrystallization from toluene then ethyl ether provided material for elemental analysis: mp 156-156.5 °C; 300 MHZ ¹H NMR (CDCl₃) 01.13 (s, 9H), 1.28 (s, 9H), 3.62 (t, 2H, J = 4.9 Hz), 3.99 (s, 3H), 4.62 (t, 2H, J = 4.9 Hz), 6.83-6.88 (m, 1H), 6.96-6.99 (d, 1H, J = 8.1 Hz), 7.08-7.13 (m, 1H), 7.29 (d, 1H, J = 8.1 Hz), 7.38-7.42 (m, 3H), 8.38 (d, 2H, J = 8.6 Hz), 8.98 (d, 2H), 9.1 (br, 1H); IR (KBr pellet) 3300-3200, 2975, 2890, 2840, 1575, 1500 cm⁻¹. Anal Calcd for C₃₁H₃₇N₅O₆S: C, 61.27; H, 6.14; N, 11.52. Found: C, 61.53; H, 6.37; N, 11.42.

### Example 5

This example illustrates the preparation of p-*tert*-butyl-N-[6-(2-formyloxy-ethoxy)-5-(O-methoxy-phenoxy) [2,2'-bipyrimidin]-4-yl] benzenesulfonamide monoethyl alcohol solvate (8).

A mixture of p-*tert*-butyl-N-[ 6-(2-*tert*-butyl-ethoxy)-5-(O-methoxy-phenoxy) [2,2'-bipyrimidin]-4-yl] benzenesulfonamide (6) (250.82 g, 0.413 mol) and 500 mL 95-97% formic acid was heated at 85 °C for 4 h. The resulting yellow solution was cooled and diluted with 800 mL toluene. Formic acid and toluene were distilled as the azeotrope using a 1000 mL distillation storage head [Ace Glass catalog # 6620-14] as a layer-separating collector at 35-39 °C and 97-102 mm Hg (collected 680 mL top phase and 450 mL bottom phase).

At this point gas chromatography (GC) analysis indicates the toluene distillate contains only trace formic acid and the product-toluene ratio (LC area %) was ~92:4. The suspension was cooled to 50 °C, diluted with 615 mL of absolute ethanol, then heated to reflux. The solution was allowed to cool to 25°C at ~150 rpm over 18 h. The resulting suspension was cooled to -5 °C, stirred for 2 h, then decanted (collected 400 mL in 75 min). The wet solid was taken up in 500 mL of absolute ethanol at reflux. The solution was allowed to cool to 25 °C at ~150 rpm over 4 h then the suspension was decanted (585 mL in 40 min). The wet solid was used directly in the next step.

Recrystallization from anhydrous ethanol provided material for elemental analysis: mp 138.5-140 °C; 300 MHZ ¹H NMR (CDCl₃) □ 1.21 (t, 3H, J = 7.0 Hz), 1.29 (s, 9H), 1.67 (br, 1H), 3.70 (m, 2H), 3.90 (s, 3H), 4.35 (m, 2H), 4.71 (m, 2H), 6.80-6.85 (m, 1H), 6.95 (d, 1H, J = 7.5 Hz), 7.03-7.11 (m, 2H), 7.40-7.44 (m, 3H), 7.89 (s, 1H), 8.41 (d, 2H, J = 8.4 Hz), 8.93 (br, 1H), 8.99 (d, 2H); IR (KBr pellet) 3600-3240, 2970, 2910, 2870, 1725, 1685, 1580, 1560 cm⁻¹. Anal Calcd for C₃₀H₃₅N₅O₈S: C, 57.59; H, 5.64; N, 11.19. Found: C, 57.40; H, 5.51; N, 11.43.

### Example 6

This example illustrates the preparation of p-*tert*-butyl-N-[6-(2-formyloxy-ethoxy)-5-(O-methoxy-phenoxy) [2,2'-bipyrimidin]-4-yl] benzenesulfonamide (7).

A mixture of p-*tert*-butyl-N-[6-(2-*tert*-butyloxy-ethoxy)-5-(O-methoxy-phenoxy) [2,2'-bipyrimidin]-4-yl] benzenesulfon-amide (6) (47.692 g, 71.84 mmol) and 78 mL of 96% formic acid was heated at 90 °C for 3 hours. The resulting yellow solution, containing some black specs, was cooled to 25 °C and the volatiles were removed on a roto-evaporator at 45 °C, and then on a vacuum pump overnight. The residual syrup was taken up in 200 mL ethyl acetate. The suspension was suction filtered, and the precipitate was washed with 50 mL ethyl acetate.

The mother liquors were concentrated on a rotary evaporator at 35 °C and the residue was triturated with 200 mL ethyl ether. The precipitate was suction filtered, then washed with 50 mL ethyl ether. The potassium formate was dried in vacuo for 20 hours at 25 °C to afford 5.11 g of colorless solid. The Bósentan formate (7) was dried in vacuo for 20 hours at 25 °C to afford 45.028 g of colorless solid.
Theoretical yield of the potassium formate: 6.044 g.
Theoretical yield of Bosentan formate (7): 41.644 g.

### Example 7

This example illustrates the preparation of Bosentan (1).

Absolute ethanol (600 mL), 165.2 g of 30% sodium hydroxide (1.239 mol NaOH), and 175 mL water were added to the wet p-*tert*-butyl-N- [6-(2-formyloxy-ethoxy)-5-(O-methoxy-phenoxy) [2,2'-bipyrimidin]-4-yl] benzenesulfonamide monoethyl alcohol solvate (8). The resulting solution was stirred at 25°C for 60 min. The suspension was slowly acidified with 77 mL of 12 N HCI to pH 5 with ice cooling to maintain 25 °C. Water (350 mL) was added dropwise then the suspension was stirred at 25 °C for 3 h. The precipitate was suction filtered, washed with 250 mL of 1:1 ethanol-water then briefly air dried at 25 °C.

### Example 8

This example illustrates the purification of Bosentan (1).

The wet crude Bosentan (1) from Example 7 was taken up in 650 mL anhydrous ethanol at reflux. Water (650 mL) was added dropwise at reflux. The resulting suspension was allowed to cool to 25 °C at ~150 rpm over 6 h. The precipitate was suction filtered and air dried at 25 °C for 16 h to afford 214.47 g of near colorless crystals (91.2% from p-tert-butyl-N-[6-(2-tert-butyloxy-ethoxy)-5-(O-methoxy-phenoxy) [2,2'-bipyrimidin]-4-yl] benzenesulfonamide (6)).

### Example 9

This example illustrates the preparation of p-*tert*-butyl-N-[6-chloro-5-(O-methoxy-phenoxy) [2,2'-bipyrimidin]-4-yl] benzenesulfonamide sodium salt.

Into a 100 mL 3-neck Morton flask with condenser, nitrogen adapter, and overhead mechanical stirrer was added 4-*tert*-butylbenzenesulfonamide (1.851 g, 8.68 mmol), 4,6-dichloro-5-(O-methoxy-phenoxy)-2,2'-bipyrimidine (3) (3.121 g, 8.93 mmol), and anhydrous sodium carbonate (2.305 g, 21.75 mmol). The flask was sealed and the atmosphere was changed to dry nitrogen through 10 nitrogen-vacuum purge cycles.

2-Methyltetrahydrofuran (30 mL) was added via syringe and the suspension was refluxed for 25 hours at an external bath temperature of 80°C. At approximately 23.5 hours, at reflux, 190 mg of tetrabutyl ammonium bromide (TBAB) was added followed by another 810 mg TBAB. Reaction progress was followed by TLC (EtOAc) for a total of 24 hours. The suspension appears identical to that formed by the corresponding potassium salt.

### Example 10

This example illustrates the precipitation of phosphate from the aqueous waste of a reaction for preparing 4,6-dichloro-5-(O-methoxy-phenoxy)-2,2'-bipyrimidine (3).

The aqueous layers from the pyrimidinedichloride (3) workup (50 g scale) were combined then filtered though 0.45 micron media to yield 650 mL of a clear, light yellow solution containing about 0.985 M phosphate (as PO₄⁻²). The filtrate (100 mL) was charged to a 500 mL flask equipped with an overhead stirrer. After calcium oxide (3, 4, or 5 equivalents) was added, the white slurry was agitated vigorously for 30 to 60 minutes at 20-22 °C and then filtered through a coarse sintered glass funnel.

The slurries produced using 3 or 4 equivalents of calcium oxide both filtered well. Soluble phosphate was reduced from over 40,000 ppm to 4 ppm using 3 equivalents of calcium oxide. Soluble phosphate was reduced to just 1 ppm using 4 equivalents of calcium oxide.

Those skilled in the art will appreciate that numerous changes and modifications may be made to the preferred embodiments of the invention. It is therefore intended that the appended claims cover all such equivalent variations as fall within the true spirit and scope of the invention.

## Claims

1. A process for preparing a 1,2-diheteroethylene sulfonamide of the formula: 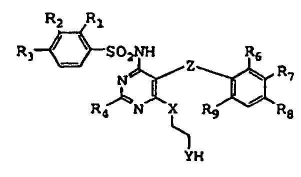
comprising:
(a) contacting a pyrimidine monohalide of the formula: 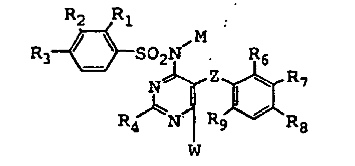
with a mono-protected 1,2-diheteroethylene anion of the formula M₁XCH₂CH₂YR₅ in an aprotic nonpolar solvent to produce a mono-protected 1,2-diheteroethylene sulfonamide of the formula: 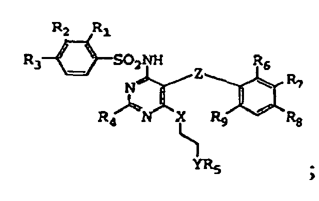 and
(b) removing R₅ group to produce said 1,2-diheteroethylene sulfonamide,
wherein
R₁ is hydrogen, lower alkyl, lower alkoxy, lower alkylthio, halogen or trifluoromethyl;
R₂ is hydrogen, halogen, lower alkoxy or trifluoromethyl; and
R₃ is hydrogen, halogen, lower alkyl, lower alkylthio, trifluoromethyl, cycloalkyl, lower alkoxy or trifluoromethoxy; or
R₂ and R₃ together can be butadienylene, methylenedioxy, ethylenedioxy or isopropylidenedioxy;
R₄ is hydrogen, lower alkyl, cycloalkyl, trifluoromethyl, lower alkoxy, lower alkylthio, lower alkylthio-lower alkyl, hydroxy-lower alkyl, hydroxy-lower alkoxy, lower alkoxy-lower alkyl, hydroxy-lower alkoxy-lower alkyl, hydroxy-lower alkoxy-lower alkoxy, lower alkylsulfinyl, lower alkylsulfonyl, 2-methoxy-3-hydroxypropoxy, 2-hydroxy-3-phenylpropyl, amino-lower alkyl, lower alkylamino-lower alkyl, di-lower alkylamino-lower alkyl, amino, lower alkyl-amino, di-lower alkylamino, arylamino, aryl, arylthio, aryloxy, aryl-lower alkyl or heterocydyl;
R₅ is a hydroxy protecting group;
R₆, R₇, R₈ and R₉ are independently hydrogen, halogen, lower alkyl, trifluoromethyl, lower alkoxy, lower alkylthio, hydroxy, hydroxymethyl, cyano, carboxyl, formyl, methylsulfinyl, methylsulfonyl, methylsulfonyloxy or lower alkyloxy-carbonyloxy; or
R₇ together with R₆ or R₈ can be butadienylene, methylenedioxy, ethylenedioxy or isopropylidenedioxy;
Z is O, S, ethylene, vinylene, C(=O), OCHR₁₀, or SCHR₁₀;
R₁₀ is hydrogen or lower alkyl;
X and Y are independently O, S or NH;
M is hydrogen, an alkaline metal or an alkaline earth metal;
M₁ is an alkaline metal or an alkaline earth metal; and
W is halide,
wherein the term "lower" denotes groups with 1-7 carbon atoms.

2. The process of Claim 1, wherein said aprotic nonpolar reaction solvent is toluene.

3. The process of Claim 1, further comprising the step of producing said pyrimidine monohalide, wherein said step comprises contacting a pyrimidine dihalide of the formula: 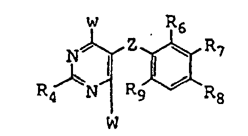 with a sulfonamide of the formula: 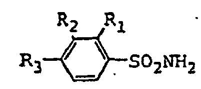
in a nonpolar solvent in the presence of a base and a phase transfer catalyst to produce said pyrimidine monohalide.

4. The process of Claim 3, wherein said base is potassium carbonate.

5. The process of Claim 3, wherein said phase transfer catalyst is selected from the group consisting of tetrabutylammonium bromide, tetrabutylphosphonium bromide, tetrabutylammonium chloride, tetrabutylphosphonium chloride, benzyltriethylammonium chloride, and tetrabutylammonium hydrogen sulfate.

6. The process of Claim 3, wherein said nonpolar solvent is toluene.

7. The process of Claim 3, wherein said pyrimidine monohalide is used in the subsequent step without isolation.

8. The process of Claim 3, further comprising the step of producing said pyrimidine dihalide, wherein said step comprises contacting a pyrimidinedione of the formula: 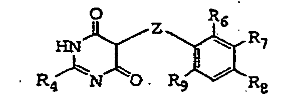
with a dehydrohalogenating agent to produce said pyrimidine dihalide.

9. The process of Claim 8, wherein said pyrimidine dihalide is used in the subsequent step without isolation.

10. The process of Claim 8, wherein said halide is chloride.

11. The process of Claim 10, wherein said dehydrohalogenating agent is selected from the group consisting of phosphorous oxychloride, phosphorous pentachloride, phosphorous trichloride, oxalyl chloride and mixtures thereof.

12. The process of Claim 1, wherein X and Y are O.

13. The process of Claim 12, wherein R₅ is *tert*-butyl,

14. The process of Claim 13, wherein said step of removing R₅ group comprises contacting said mono-protected 1,2-diheteroethylene sulfonamide with an acid.

15. The process of Claim 14, wherein said acid is formic acid.

16. The process of Claim 15, wherein said step of contacting said mono-protected 1,2-diheteroethylene sulfonamide with said formic acid produces an intermediate mono-protected 1,2-diheteroethylene sulfonamide of the formula:

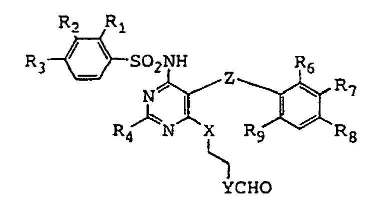

17. The process of Claim 16, further comprising contacting said intermediate mono-protected 1,2-diheteroethylene sulfonamide with a base to produce said 1,2-diheteroethylene sulfonamide.

18. A process for preparing an ethylene glycol sulfonamide of the formula: 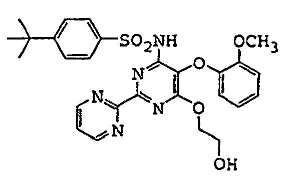
comprising:
(a) contacting a pyrimidinedione of the formula: 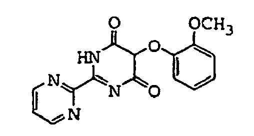
with a dehydrohalogenating agent to produce a pyrimidine dihalide of the formula: 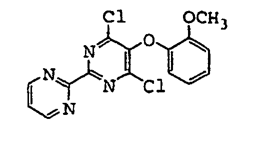
(b) contacting said pyrimidine dihalide with a sulfonamide of the formula: 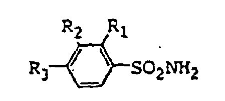
in a nonpolar aprotic solvent in the presence of a first base and a phase transfer catalyst to produce a pyrimidine monohalide of the formula: 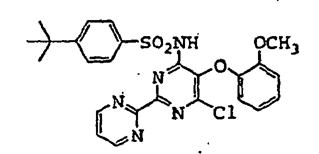
(c) contacting said pyrimidine monohalide with a mono-protected ethylene glycol of the formula HOCH₂CH₂OR₅ in said nonpolar aprotic solvent in the presence of a second base to produce a mono-protected ethylene glycol sulfonamide of the formula: 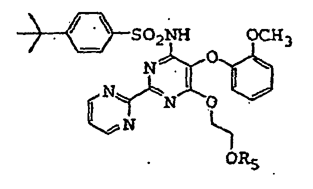
wherein
R₅ is a hydroxy protecting group; and
(d) removing the protecting group to produce said ethylene glycol sulfonamide.

19. The process of Claim 18, wherein said dehydrohalogenating agent is selected from the group consisting of phosphorous oxychloride, phosphorous pentachloride, phosphorous trichloride, oxalyl chloride and mixtures thereof.

20. The process of Claim 18, wherein said first base is potassium carbonate.

21. The process of Claim 18, wherein said first base is present in the amount of from about 1 equiv. to about 2 equiv.

22. The process of Claim 18, wherein said phase transfer catalyst is selected from the group consisting of tetrabutylammonium bromide, tetrabutylphosphonium bromide, tetrabutylammonium chloride, tetrabutylphosphonium chloride, benzyltriethylammonium chloride, and tetrabutylammonium hydrogen sulfate.

23. The process of Claim 18, wherein said phase transfer catalyst is present in the amount of from about 0.5 mole% to about 10 mole%.

24. The process of Claim 18, wherein said second base is sodium hydroxide.

25. The process of Claim 18, wherein said nonpolar aprotic solvent is toluene.

26. The process of Claim 18, wherein said R₅ is *tert*-butyl.

27. The process of Claim 26, wherein said step of removing the protecting group comprises:
(e) contacting said mono-protected ethylene glycol sulfonamide with formic acid; and
(f) contacting the resulting product of said step (e) with a third base to produce said ethylene glycol sulfonamide.

28. The process of Claim 27, wherein said third base is sodium hydroxide.

29. The process of Claim 18, wherein said steps (a)-(c) are conducted without any isolation of each resulting compounds.

30. A compound selected from the group consisting of p-*tert*-butyl-N-[6-(2-*tert-*butoxyethoxy)-5-(o-methoxyphenoxy)-2-(pyrimidin-2-yl)-pyrimidin-4-yl] benzenesulfonamide, p-*tert*-butyl-N-[6-(2-formyloxyethoxy)-5-(o-methoxyphenoxy)-2-(pyrimidin-2-yl)-pyrimidin-4-yl] benzenesulfonamide, p-*tert*-butyl-N-[6-(2-formyloxyethoxy)-5-(o-methoxyphenoxy)-2-(pyrimidin-2-yl)-pyrimidin-4-yl] benzenesulfonamide monoethyl alcohol solvate in a crystalline form.

31. The process of claim 1, wherein R₂ is hydrogen.

## Patentansprüche

1. Verfahren zur Herstellung eines 1,2-Diheteroethylensulfonamids der Formel: 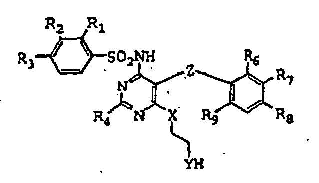
umfassend:
(a) in Kontakt bringen eines Pyrimidinmonohalogenids der Formel: 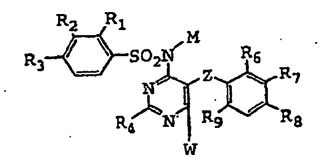
mit einem mono-geschützten 1,2-Diheteroethylenanion der Formel M₁XCH₂CH₂YR₅ in einem aprotischen unpolaren Lösungsmittel, um ein mono-geschütztes 1,2-Diheteroethylensulfonamid der Formel: 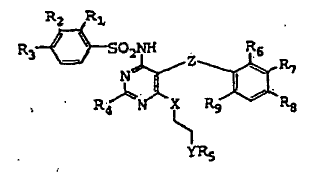
herzustellen; und
(b) Entfernen des Restes R₅, um das 1,2-Diheteroethylensulfonamid herzustellen, wobei
R₁ für Wasserstoff, Niederalkyl, Niederalkoxy, Niederalkylthio, Halogen oder Trifluormethyl steht;
R₂ für Wasserstoff, Halogen, Niederalkoxy oder Trifluormethyl steht; und
R₃ für Wasserstoff, Halogen, Niederalkyl, Niederalkylthio, Trifluormethyl, Cycloalkyl, Niederalkoxy oder Trifluormethoxy steht; oder
R₂ und R₃ zusammen Butadienylen, Methylendioxy, Ethylendioxy oder Isopropylidendioxy sein können;
R₄ für Wasserstoff, Niederalkyl, Cycloalkyl, Trifluormethyl, Niederalkoxy, Niederalkylthio, Niederalkylthioniederalkyl, Hydroxyniederalkyl, Hydroxyniederalkoxy, Niederalkoxyniederalkyl, Hydroxyniederalkoxyniederalkyl, Hydroxyniederalkoxyniederalkoxy, Niederalkylsulfmyl, Niederalkylsulfonyl, 2-Methoxy-3-hydroxypropoxy, 2-Hydroxy-3-phenylpropyl, Aminoniederalkyl, Niederalkylaminoniederalkyl, Diniederalkylaminoniederalkyl, Amino, Niederalkylamino, Diniederalkylamino, Arylamino, Aryl, Arylthio, Aryloxy, Arylniederalkyl oder Heterocyclyl steht;
R₅ eine Hydroxyschutzgruppe darstellt;
R₆, R₇, R₈ und R₉ unabhängig für Wasserstoff, Halogen, Niederalkyl, Trifluormethyl, Niederalkoxy, Niederalkylthio, Hydroxy, Hydroxymethyl, Cyano, Carboxyl, Formyl, Methylsulfinyl, Methylsulfonyl, Methylsulfonyloxy oder Niederalkyloxycarbonyloxy steht; oder
R₇ zusammen mit R₆ oder R₈ Butadienylen, Methylendioxy, Ethylendioxy oder Isopropylidendioxy sein kann;
Z für O, S, Ethylen, Vinylen, C(=O), OCHR₁₀ oder SCHR₁₀ steht;
R₁₀ für Wasserstoff oder Niederalkyl steht;
X und Y unabhängig O, S oder NH darstellen;
M für Wasserstoff, ein Alkalimetal oder ein Erdalkalimetall steht;
M₁ für ein Alkalimetall oder ein Erdalkalimetall steht; und
W für ein Halogenid steht,
wobei der Begriff "Nieder" Reste mit 1 bis 7 Kohlenstoffatomen bezeichnet.

2. Verfahren nach Anspruch 1, wobei das aprotische unpolare Reaktionslösungsmittel Toluol ist.

3. Verfahren nach Anspruch 1, weiterhin umfassend den Schritt des Herstellens des Pyrimidinmonohalogenids, wobei der Schritt das in Kontakt bringen eines Pyrimidindihalogenids der Formel: 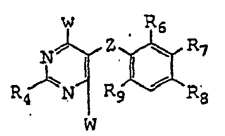
mit einem Sulfonamid der Formel: 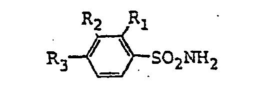
in einem unpolaren Lösungsmittel in Gegenwart einer Base und eines Phasen-Transfer-Katalysators, um das Pyrimidinmonohalogenid herzustellen, umfasst.

4. Verfahren nach Anspruch 3, wobei die Base Kaliumcarbonat ist.

5. Verfahren nach Anspruch 3, wobei der Phasen-Transfer-Katalysator ausgewählt ist aus Tetrabutylammoniumbromid, Tetrabutylphosphoniumbromid, Tetrabutylammoniumchlorid, Tetrabutylphosphoniumchlorid, Benzyltriethylammoniumchlorid und Tetrabutylammoniumwasserstoffsulfat.

6. Verfahren nach Anspruch 3, wobei das unpolare Lösungsmittel Toluol ist.

7. Verfahren nach Anspruch 3, wobei das Pyrimidininonohalogenid im nachfolgenden Schritt ohne Isolierung verwendet wird.

8. Verfahren nach Anspruch 3, weiterhin umfassend den Schritt des Herstellens des Pyrimidindihalogenids, wobei der Schritt das in Kontakt bringen eines Pyrimidindions der Formel: 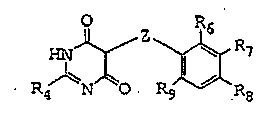
mit einem Dehydrohalogenierungsmittel, um das Pyrimidindihalogenid herzustellen, umfasst.

9. Verfahren nach Anspruch 8, wobei das Pyrimidindihalogenid im nachfolgenden Schritt ohne Isolierung verwendet wird.

10. Verfahren nach Anspruch 8, wobei das Halogenid Chlorid ist.

11. Verfahren nach Anspruch 10, wobei das Dehydrohalogenierungsmittel ausgewählt ist aus Phosphoroxychlorid, Phosphorpentachlorid, Phosphortrichlorid, Oxalylchlorid und Gemischen davon.

12. Verfahren nach Anspruch 1, wobei X und Y für O stehen.

13. Verfahren nach Anspruch 12, wobei R₅ für *tert*-Butyl steht.

14. Verfahren nach Anspruch 13, wobei der Schritt des Entfernens des Rests R₅ das in Kontakt bringen des mono-geschützten 1,2-Diheteroethylensulfonamids mit Säure umfasst.

15. Verfahren nach Anspruch 14, wobei die Säure Ameisensäure ist.

16. Verfahren nach Anspruch 15, wobei der Schritt des in Kontaktbringens des mono-geschützten 1,2-Diheteroethylensulfonamids mit der Ameisensäure ein mono-geschütztes 1,2-Diheteroethylensulfonamid-Zwischenprodukt der Formel: 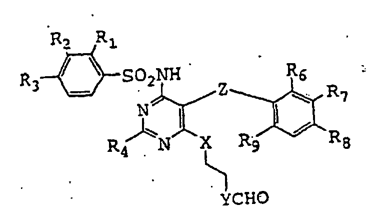
bildet.

17. Verfahren nach Anspruch 16, weiterhin umfassend das in Kontakt bringen des mono-geschützten 1,2-Diheteroethylensulfonamid-Zwischenprodukts mit einer Base, um das 1,2-Diheteroethylensulfonamid herzustellen.

18. Verfahren zur Herstellung eines Ethylenglycolsulfonamids der Formel: 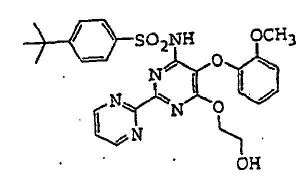
umfassend:
(a) in Kontakt bringen eines Pyrimidindions der Formel: 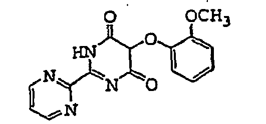
mit einem Dehydrohalogenierungsmittel, um ein Pyrimidindihalogenid der Formel: 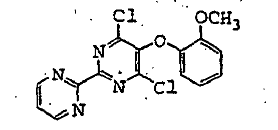
herzustellen;
(b) in Kontakt bringen des Pyrimidindihalogenids mit einem Sulfonamid der Formel: 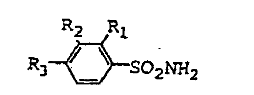
in einem unpolaren aprotischen Lösungsmittel in Gegenwart einer ersten Base und eines Phasen-Transfer-Katalysators, um ein Pyrimidinmonohalogenid der Formel: 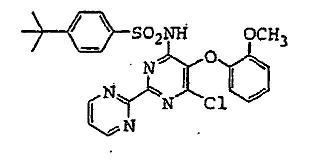
herzustellen;
(c) in Kontakt bringen des Pyrimidinmonohalogenids mit einem mono-geschützten Ethylenglycol der Formel HOCH₂CH₂OR₅ in dem unpolaren aprotischen Lösungsmittel in Gegenwart einer zweiten Base, um ein mono-geschütztes Ethylenglycolsulfonamid der Formel: 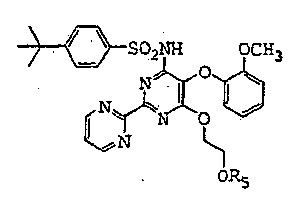
herzustellen, wobei
R₅ eine Hydroxyschutzgruppe ist; und
(d) Entfernen der Schutzgruppe, um das Ethylenglycolsulfonamid herzustellen.

19. Verfahren nach Anspruch 18, wobei das Dehydrohalogenierungsmittel ausgewählt ist aus Phosphoroxychlorid, Phosphorpentachlorid, Phosphortrichlorid, Oxalylchlorid und Gemischen davon.

20. Verfahren nach Anspruch 18, wobei die erste Base Kaliumcarbonat ist.

21. Verfahren nach Anspruch 18, wobei die erste Base in einer Menge von etwa 1 Äquivalent bis etwa 2 Äquivalenten vorhanden ist.

22. Verfahren nach Anspruch 18, wobei der Phasen-Transfer-Katalysator ausgewählt ist aus Tetrabutylammoniumbromid, Tetrabutylphosphoniumbromid, Tetrabutylammoniumchlorid, Tetrabutylphosphoniumchlorid, Benzyltriethylammoniumchlorid und Tetrabutylammoniumwasserstoffsulfat.

23. Verfahren nach Anspruch 18, wobei der Phasen-Transfer-Katalysator in einer Menge von etwa 0,5 Mol.-% bis etwa 10 Mol.-% vorhanden ist.

24. Verfahren nach Anspruch 18, wobei die zweite Base Natriumhydroxid ist.

25. Verfahren nach Anspruch 18, wobei das unpolare aprotische Lösungsmittel Toluol ist.

26. Verfahren nach Anspruch 18, wobei R₅ *tert*-Butyl ist.

27. Verfahren nach Anspruch 26, wobei der Schritt des Entfemens der Schutzgruppe Folgendes umfasst:
(e) in Kontakt bringen des mono-geschützten Ethylenglycolsulfonamids mit Ameisensäure; und
(f) in Kontakt bringen des resultierenden Produkts aus Schritt (e) mit einer dritten Base, um das Ethylenglycolsulfonamid herzustellen.

28. Verfahren nach Anspruch 27, wobei die dritte Base Natriumhydroxid ist.

29. Verfahren nach Anspruch 18, wobei die Schritte (a) bis (c) ohne jegliche Isolierung der jeweils resultierenden Verbindungen durchgeführt werden.

30. Verbindung ausgewählt aus p-*tert*-Butyl-N-[6-(2-*tert*-butoxyethoxy)-5-(o-methoxyphenoxy)-2-(pyrimidin-2-yl)-pyrimidin-4-yl]benzolsulfonamid, p-*tert*-Butyl-N-[6-(2-formyloxyethoxy)-5-(o-methoxyphenoxy)-2-(pyrimidin-2-yl)-pyrimidin-4-yl]benzolsulfonamid, p-*tert*-Butyl-N-[6-(2-formyloxyethoxy)-5-(o-methoxyphenoxy)-2-(pyrimidin-2-yl)-pyrimidin-4-yl]benzolsulfonamidmonoethylalkoholsolvat in kristalliner Form.

31. Verfahren nach Anspruch 1, wobei R₂ für Wasserstoff steht.

## Revendications

1. Procédé pour la préparation d'un 1,2-dihétéroéthylène-sulfonamide de formule : 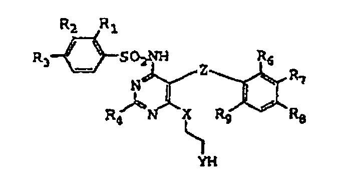
comprenant :
(a) la mise en contact d'un monohalogénure de pyrimidine de formule : 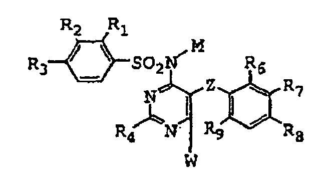
avec un anion 1,2-dihétéroéthylénique mono-protégé de formule M₁XCH₂CH₂YR₅ dans un solvant aprotique non polaire, pour l'obtention d'un 1,2-dihétéroéthylène-sulfonamide mono-protégé de formule : 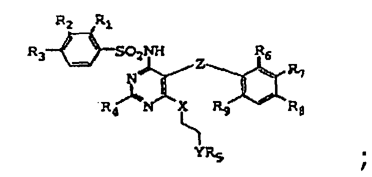 et
(b) l'élimination du groupe R₅, pour l'obtention dudit 1,2-dihétéroéthylène-sulfonamide,
formules dans lesquelles
R₁ est un atome d'hydrogène ou d'halogène ou un groupe alkyle inférieur, alcoxy inférieur, (alkyl inférieur)thio ou trifluorométhyle ;
R₂ est un atome d'hydrogène ou d'halogène ou un groupe alcoxy inférieur ou trifluorométhyle ; et
R₃ est un atome d'hydrogène ou d'halogène ou un groupe alkyle inférieur, (alkyl inférieur)thio, trifluorométhyle, cycloalkyle, alcoxy inférieur ou trifluorométhoxy ; ou
R₂ et R₃ peuvent former ensemble un groupe butadiénylène, méthylènedioxy, éthylènedioxy ou isopropylidènedioxy ;
R₄ est un atome d'hydrogène ou un groupe alkyle inférieur, cycloalkyle, trifluorométhyle, alcoxy inférieur, (alkyl inférieur)thio, (alkyl inférieur)thio-alkyle inférieur, hydroxyalkyle inférieur, hydroxyalcoxy inférieur, (alcoxy inférieur)-alkyle inférieur, hydroxy(alcoxy inférieur)-alkyle inférieur, hydroxy(alcoxy inférieur)-alcoxy inférieur, (alkyl inférieur)sulfinyle, (alkyl inférieur)sulfonyle, 2-méthoxy-3-hydroxypropoxy, 2-hydroxy-3-phénylpropyle, aminoalkyle inférieur, (alkyl inférieur)amino-alkyle inférieur, di(alkyl inférieur)amino-alkyle inférieur, amino, (alkyl inférieur)amino, di(alkyl inférieur)amino, arylamino, aryle, arylthio, aryloxy, aryl(alkyle inférieur) ou hétérocyclyle ;
R₅ est un groupe protecteur de fonction hydroxy ;
R₆, R₇, R₈ et R₉ représentent indépendamment un atome d'hydrogène ou d'halogène ou un groupe alkyle inférieur, trifluorométhyle, alcoxy inférieur, (alkyl inférieur)thio, hydroxy, hydroxyméthyle, cyano, carboxy, formyle, méthylsulfinyle, méthylsulfonyle, méthylsulfonyloxy ou (alkyl inférieur)oxy-carbonyloxy ; ou
R₇, conjointement avec R₆ ou R₈, peut représenter un groupe butadiénylène, méthylènedioxy, éthylènedioxy ou isopropylidènedioxy ;
Z est O, S, un groupe éthylène, vinylène, C(=O), OCHR₁₀ ou SCHR₁₀;
R₁₀ est un atome d'hydrogène ou un groupe alkyle inférieur ;
X et Y représentent indépendamment O, S ou NH ;
M est un atome d'hydrogène ou de métal alcalin ou alcalino-terreux ;
M₁ est un métal alcalin ou un métal alcalino-terreux ; et
W est un halogénure,
le terme "inférieur" désignant des groupes ayant de 1 à 7 atomes de carbone.

2. Procédé selon la revendication 1, dans lequel ledit solvant aprotique non polaire utilisé dans la réaction est le toluène.

3. Procédé selon la revendication 1, comprenant en outre l'étape de production dudit monohalogénure de pyrimidine, ladite étape comprenant la mise en contact d'un dihalogénure de pyrimidine de formule : 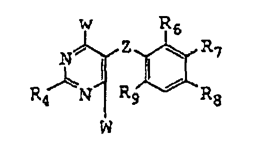 avec un sulfonamide de formule: 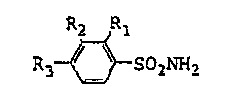
dans un solvant non polaire, en présence d'une base et d'un catalyseur de transfert de phase, pour l'obtention dudit monohalogénure de pyrimidine.

4. Procédé selon la revendication 3, dans lequel ladite base est le carbonate de potassium.

5. Procédé selon la revendication 3, dans lequel ledit catalyseur de transfert de phase est choisi dans l'ensemble constitué par le bromure de tétrabutylammonium, le bromure de tétrabutylphosphonium, le chlorure de tétrabutylammonium, le chlorure de tétrabutylphosphonium, le chlorure de benzyltriéthylammonium et l'hydrogénosulfate de tétrabutylammonium.

6. Procédé selon la revendication 3, dans lequel ledit solvant non polaire est le toluène.

7. Procédé selon la revendication 3, dans lequel ledit monohalogénure de pyrimidine est utilisé dans l'étape suivante sans être isolé.

8. Procédé selon la revendication 3, comprenant en outre l'étape de production dudit dihalogénure de pyrimidine, dans lequel ladite étape comprend la mise en contact d'une pyrimidinedione de formule : 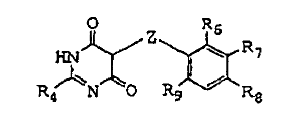
avec un agent de déshydrohalogénation, pour l'obtention dudit dihalogénure de pyrimidine.

9. Procédé selon la revendication 8, dans lequel ledit dihalogénure de pyrimidine est utilisé dans l'étape suivante sans être isolé.

10. Procédé selon la revendication 8, dans lequel ledit halogénure est le chlorure.

11. Procédé selon la revendication 10, dans lequel ledit agent de déshydrohalogénation est choisi dans l'ensemble constitué par l'oxychlorure de phosphore, le pentachlorure de phosphore, le trichlorure de phosphore, le chlorure d'oxalyle et des mélanges de ceux-ci.

12. Procédé selon la revendication 1, dans lequel X et Y représentent O.

13. Procédé selon la revendication 12, dans lequel R₅ est le groupe tert-butyle.

14. Procédé selon la revendication 13, dans lequel ladite étape d'élimination du groupe R₅ comprend la mise en contact dudit 1,2-dihétéroéthylène-sulfonamide mono-protégé avec un acide.

15. Procédé selon la revendication 14, dans lequel ledit acide est l'acide formique.

16. Procédé selon la revendication 15, dans lequel ladite étape de mise en contact dudit 1,2-dihétéroéthylène-sulfonamide mono-protégé avec ledit acide formique donne un 1,2-dihétéroéthylène-sulfonamide mono-protégé intermédiaire de formule :

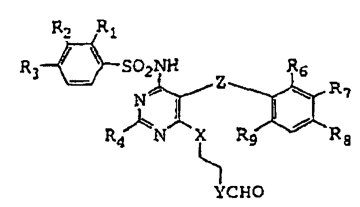

17. Procédé selon la revendication 16, comprenant en outre la mise en contact dudit 1,2-dihétéroéthylène-sulfonamide mono-protégé intermédiaire avec une base, pour l'obtention dudit 1,2-dihétéroéthylène-sulfonamide.

18. Procédé pour la préparation d'un éthylèneglycol-sulfonamide de formule : 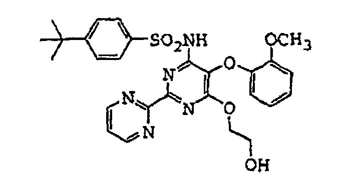
comprenant :
(a) la mise en contact d'une pyrimidinedione de formule : 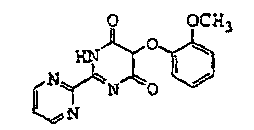
avec un agent de déshydrohalogénation, pour l'obtention d'un dihalogénure de pyrimidine de formule : 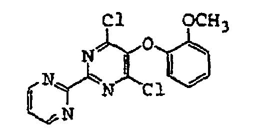
(b) la mise en contact dudit dihalogénure de pyrimidine avec un sulfonamide de formule : 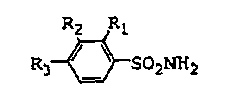
dans un solvant aprotique non polaire, en présence d'une première base et d'un catalyseur de transfert de phase, pour l'obtention d'un monohalogénure de pyrimidine de formule : 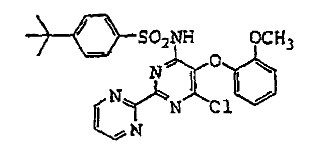
(c) la mise en contact dudit monohalogénure de pyrimidine avec un éthylèneglycol mono-protégé de formule HOCH₂CH₂OR₅ dans ledit solvant aprotique non polaire, en présence d'une seconde base, pour l'obtention d'un éthylèneglycol-sulfonamide mono-protégé de formule : 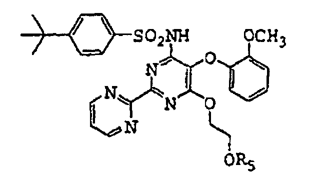
dans laquelle
R₅ est un groupe protecteur de fonction hydroxy ; et
(d) l'élimination du groupe protecteur pour l'obtention dudit éthylèneglycol-sulfonamide.

19. Procédé selon la revendication 18, dans lequel ledit agent de déshydrohalogénation est choisi dans l'ensemble constitué par l'oxychlorure de phosphore, le pentachlorure de phosphore, le trichlorure de phosphore, le chlorure d'oxalyle et des mélanges de ceux-ci.

20. Procédé selon la revendication 18, dans lequel ladite première base est le carbonate de potassium.

21. Procédé selon la revendication 18, dans lequel ladite première base est présente en une quantité d'environ 1 équivalent à environ 2 équivalents.

22. Procédé selon la revendication 18, dans lequel ledit catalyseur de transfert de phase est choisi dans l'ensemble constitué par le bromure de tétrabutylammonium, le bromure de tétrabutylphosphonium, le chlorure de tétrabutylammonium, le chlorure de tétrabutylphosphonium, le chlorure de benzyltriéthylammonium et l'hydrogénosulfate de tétrabutylammonium.

23. Procédé selon la revendication 18, dans lequel ledit catalyseur de transfert de phase est présent en une quantité d'environ 0,5 % en moles à environ 10 % en moles.

24. Procédé selon la revendication 18, dans lequel ladite seconde base est l'hydroxyde de sodium.

25. Procédé selon la revendication 18, dans lequel ledit solvant aprotique non polaire est le toluène.

26. Procédé selon la revendication 18, dans lequel ledit radical R₅ est le groupe tert-butyle.

27. Procédé selon la revendication 26, dans lequel ladite étape d'élimination du groupe protecteur comprend :
(e) la mise dudit éthylèneglycol-sulfonamide mono-protégé en contact avec de l'acide formique ; et
(f) la mise du produit résultant de l'étape (e) en contact avec une troisième base, pour l'obtention dudit éthylèneglycol-sulfonamide.

28. Procédé selon la revendication 27, dans lequel ladite troisième base est l'hydroxyde de sodium.

29. Procédé selon la revendication 18, dans lequel lesdites étapes (a)-(c) sont effectuées sans aucun isolement de chaque composé résultant.

30. Composé choisi dans l'ensemble constitué par le p-tert-butyl-N-[6-(2-tert-butoxyéthoxy)-5-(o-méthoxyphénoxy)-2-(pyrimidin-2-yl)pyrimidin-4-yl]benzènesulfonamide, le p-tert-butyl-N-[6-(2-formyloxyéthoxy)-5-(o-méthoxyphénoxy)-2-(pyrimidin-2-yl)pyrimidin-4-yl]benzène-sulfonamide, le produit de solvatation de p-tert-butyl-N-[6-(2-formyloxyéthoxy)-5-(o-méthoxyphénoxy)-2-(pyrimidin-2-yl)pyrimidin-4-yl]benzènesulfonamide et d'alcool monoéthylique sous une forme cristalline.

31. Procédé selon la revendication 1, dans lequel R₂ est un atome d'hydrogène.
